# EUROPÄISCHE PATENTSCHRIFT

(11) **EP 2 794 022 B1**
(45) Veröffentlichungstag und Bekanntmachung des Hinweises auf die Patenterteilung: **02.01.2019**
(21) Anmeldenummer: 12805613.2
(22) Anmeldetag: 22.11.2012
(51) Int. Cl.: A61Q 5/06, A61K 8/35, A61K 8/41, A61K 8/55

(54) **FÄRBEMITTEL MIT KATIONISCHEN DIREKTZIEHENDEN FARBSTOFFEN UND LECITHINE**
COLORING AGENT COMPRISING CATIONIC DIRECT DYES AND LECITHINS
COMPOSITION DE COLORATION CONTENANT DES COLORANTS DIRECTS CATIONIQUES ET DES LÉCITHINES

(30) Priorität: 20.12.2011 DE 102011089218
(43) Veröffentlichungstag der Anmeldung: 29.10.2014
(73) Patentinhaber: Henkel AG & Co. KGaA, 40589 Düsseldorf (DE)
(72) Erfinder: WESER, Gabriele, 41472 Neuss (DE); KOLONKO, Claudia, 42857 Remscheid (DE)
(86) Internationale Anmeldenummer: PCT/EP2012/073287
(87) Internationale Veröffentlichungsnummer: WO 2013/092092

(56) Entgegenhaltungen:
- EP-A1- 1 820 826
- EP-A1- 2 329 809
- DE-A1- 10 060 814
- DE-A1-102004 028 465

## Beschreibung

Die vorliegende Erfindung betrifft Mittel zum Färben und gegebenenfalls Aufhellen von keratinischen Fasern, insbesondere menschlichen Haaren, welche bestimmte kationische Anthrachinonfarbstoffe und Lecithine enthalten. Weiterhin wird die Verwendung dieser Mittel zur Erzeugung von Haarfärbungen mit erhöhtem Glanz, intensivem Farbergebnis, verbesserten Echtheitseigenschaften sowie verringerter Selektivität beschrieben.

Für das Färben von keratinischen Fasern kommen im allgemeinen entweder direktziehende Farbstoffe oder Oxidationsfarbstoffe zur Anwendung. Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Des Weiteren können einige Oxidationsfarbstoffvorprodukte bzw. bestimmte Mischungen von Oxidationsfarbstoffvorprodukten bei Personen mit empfindlicher Haut sensibilisierend wirken. Direktziehende Farbstoffe werden unter schonenderen Bedingungen appliziert. Ihr Nachteil liegt jedoch darin, dass die Färbungen häufig nur über unzureichende Echtheitseigenschaften verfügen, insbesondere bei der Haarwäsche, aber auch gegenüber äußeren Einflüssen, wie Sonnenlicht oder reaktiven Umweltchemikalien, wie beispielsweise Schwimmbadwasser. Solche Färbungen sind auch gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Eine besondere Herausforderung für die Haarfärbung mit direktziehenden Farbstoffen stellt die gleichmäßige Färbung von häufig vorbehandeltem Haar, wie beispielsweise gebleichtem oder dauergewelltem Haar, dar, bei dem die Faser in den verschiedenen Längen oder verschieden behandelten Bereichen eine sehr unterschiedlich starke Vorschädigung besitzt. Während der Färbung selbst kann das Färbemittel auf unterschiedlich vorgeschädigtem Haar ein ungleichmäßiges Färbeverhalten zeigen, aber auch durch wiederholte Haarwäsche können die Farbstoffe in den unterschiedlichen Haarbereichen verschieden stark auswaschen werden, wodurch ein uneinheitliches und damit unerwünschtes Farbergebnis erzielt wird.

Die Herstellung von Färbeformulierungen mit verringerter Selektivität, mittels welcher auf den unterschiedlich stark geschädigten Partien der Haare ein einheitliches Farbergebnis erzielt werden kann, steht bei der Entwicklung Färbeprodukten auf Basis von Direktziehern nach wie vor besonders im Fokus. Insbesondere soll diese verringerte Selektivität nicht nur direkt nach dem Färbeprozess, sondern auch nach wiederholten Haarwäschen noch vorhanden sein.

Die Aufgabe der vorliegenden Erfindung ist daher die Bereitstellung eines Färbemittels für keratinische Fasern, insbesondere menschliche Haare, welches neben anderen positiven Echtheitseigenschaften insbesondere eine geringe Selektivität (bzw. ein gutes Egalisiervermögen) und eine gute Waschechtheit besitzt.

Die mit den erfindungsgemäßen Mitteln erzeugten Färbungen sollen sowohl direkt nach dem Färbevorgang als auch nach wiederholten Haarwäschen ein brillantes und intensives Farbergebnis liefern. Hierbei sollen die Haare nach Anwendung des Färbemittels auch bei Vorhandensein von Haaren unterschiedlichen Schädigungsgrades gleichmäßig gefärbt sein, wobei diese Gleichmäßigkeit im Farbergebnis auch nach wiederholten Haarwäschen noch vorhanden sein soll. Insbesondere sollen brillante und neutrale Blaunuancen bzw. Nuancen im Blaubereich mit den oben beschriebenen vorteilhaften Echtheitseigenschaften erzielt werden, welche sich hervorragend auch für die Mattierung eigenen. Zusätzlich sollen die Mittel eine sowohl im Hinblick auf den Anwendungsprozess als auch auf die Färbeleistung optimierte Viskosität besitzen.

Der Einsatz von kationischen Anthrachinonfarbstoffen in Produkten zum Färben von keratinischen Fasern ist prinzipiell bereits aus dem Stand der Technik, beispielsweise aus EP 1 006 154 B1 oder aus EP 1 820 826 A1, bekannt. Ferner werden in EP 2 329 809 Kombinationen von kationischen Anthrachinonfarbstoffen mit Oxidationsfarbstoffvorprodukten vom Entwicklertyp für die oxidative Färbung von Haaren beansprucht.

Überraschend hat sich gezeigt, dass Kombinationen aus bestimmten kationischen Anthrachinonen als direktziehenden Farbstoffen mit Lecithin zu Färbungen führen, welche die Aufgabenstellung in herausragendem Maß lösen.

Die Kombinationen aus den bestimmten kationischen Anthrachinone gemäß erstem Erfindungsgegenstand mit Lecithin sind im Stand der Technik bislang noch nicht beschrieben.

Ein erster Gegenstand der vorliegenden Erfindung ist ein Mittel zum Färben und gegebenenfalls Aufhellen von keratinischen Fasern, insbesondere menschlichen Haaren, welches dadurch gekennzeichnet ist, dass es in einem kosmetischen Träger
(a) mindestens eine Verbindung der Formel (I) 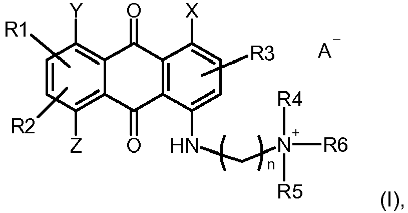 worin
   - R1, R2, R3: jeweils unabhängig voneinander für Wasserstoff, Halogen, eine Nitrogruppe, eine Cyanogruppe, eine Carboxylgruppe, eine Sulfonsäuregruppe, eine Hydroxygruppe, eine C₁-C₆-Acylaminogruppe, eine Carboxamidgruppe, eine Sulfonamidgruppe, eine C₁-C₆-Alkylgruppe, C₁-C₆-Alkoxygruppe, eine C₂-C₆-Alkenylgruppe, eine C₂-C₆-Hydroxyalkyl gruppe oder eine C₁-C₆-Alkoxy-C₂-C₆-alkylgruppe stehen;
   - R4, R5, R6: jeweils unabhängig voneinander für eine C₁-C₆-Alkylgruppe, eine C₂-C₆-Alkenyl-gruppe, für eine C₂-C₆-Hydroxyalkylgruppe oder eine C₁-C₆-Alkoxy-C₂-C₆-alkylgruppe stehen;
   oder R4 und R5 bilden zusammen mit dem quartären Stickstoffatom, an das sie gebunden sind, einen 5-, 6- oder 7-gliedrigen Ring, der gegebenenfalls weitere Heteroatome enthalten kann und/oder weitere Substituenten tragen kann;
   - X, Y, Z: jeweils unabhängig voneinander für Wasserstoff, eine Hydroxygruppe oder eine Gruppe N(R7)(R8) stehen,
   worin
   R7 und R8 jeweils unabhängig voneinander für Wasserstoff, eine C₁-C₆-Alkylgruppe, eine C₂-C₆-Alkenylgruppe, für eine C₂-C₆-Hydroxyalkylgruppe oder eine C₁-C₆-Alkoxy-C₂-C₆-alkylgruppe stehen,
   unter der Maßgabe, dass mindestens einer der Reste X, Y, Z für eine Gruppe N(R7)(R8) steht,
   - n: für eine Zahl von 2 bis 6 steht,
   - A⁻: für ein physiologisch verträgliches Anion steht, und
(b) mindestens ein Lecithin enthält.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Der erfindungsgemäß verwendete Begriff "Färben von Keratinfasern" umfasst jedwede Form der Farbveränderung der Fasern. Umfasst sind insbesondere die unter den Begriffen Tönung, Aufhellung, Blondierung, Bleiche, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise färbende Blondierungen.

Die erfindungsgemäßen Mittel enthalten die Wirkstoffe in einem kosmetischen Träger, bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind. Es ist aber auch denkbar, die erfindungsgemäßen Mittel in eine pulverförmige oder auch tablettenförmige Formulierung zu integrieren.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel. wässriger Träger enthält im Sinne der Erfindung mindestens 30 Gew.-%, insbesondere mindestens 50 Gew.-% Wasser, bezogen auf das Gesamtgewicht der Mittel. Erfindungsgemäß bevorzugt sind wässrige Träger, so dass das Mittel einen Anteil von mindestens 80 Gew.-%, bevorzugt mindestens 85 Gew.-%, bezogen auf das Gesamtgewicht des Mittels aufweist.

Als ersten wesentlichen Inhaltsstoff (a) enthalten die erfindungsgemäßen Mittel mindestens einen direktziehenden kationischen Anthrachinonfarbstoff der allgemeinen Formel (I).

Die Substituenten R1 bis R8 der Verbindung der Formel (I) sind nachfolgend beispielhaft erläutert: Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine C₁-C₆-Hydroxyalkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Erfindungsgemäß bevorzugte C₁-C₆-Alkoxygruppen sind die Methoxy- oder die Ethoxygruppe. Beispiele für Halogenatome sind F-, Cl-, Br- oder I-Atome, wobei Br- oder Cl-Atome ganz besonders bevorzugt sind. Bevorzugte Beispiele für erfindungsgemäße C₁-C₆-Alkoxy-C₂-C₆-alkylgruppen sind die Methoxyethyl-, Ethoxyethyl-, Methoxypropyl-, Methoxybutyl-, Ethoxypropyl-, Ethoxybutyl- und die Methoxyhexylgruppe. Beispiele für eine C₁-C₆-Acylaminogruppe sind die Acetamidgruppe, die Propanamidgruppe und die Butanamidogruppe, wobei die Acetamidgruppe bevorzugt ist. Als bevorzugte Beispiele für einen 5-, 6- oder 7-gliedrigen Ring, der aus R4, R5 und dem quartären Stickstoffatom gebildet wird, sind der Pyrrolidiniumring, der Piperidiniumring, der Morpholiniumring und der 1-Azepaniumring zu nennen.

Farbstoffe der Formel (I), in denen R1, R2 und R3 unabhängig voneinander für Wasserstoff, Halogen, eine Carboxylgruppe, eine Sulfonsäuregruppe, eine C₁-C₆-Alkylgruppe oder eine C₁-C₆-Alkoxygruppe stehen, liefern besonders intensive Färbeergebnisse und sind daher bevorzugt.

Weiterhin bevorzugt ist es, wenn einer der Reste ausgewählt aus R1, R2 und R3 für Halogen, eine Carboxylgruppe, eine Sulfonsäuregruppe, eine C₁-C₆-Alkylgruppe oder eine C₁-C₆-Alkoxygruppe steht und die anderen beiden Reste ausgewählt aus R1, R2 und R3 beide für Wasserstoff stehen.

Eine bevorzugte Ausführungsform des ersten Erfindungsgegenstands ist ein Mittel zum Färben und gegebenenfalls Aufhellen von keratinischen Fasern, welches dadurch gekennzeichnet ist, dass es eine Verbindung der Formel (I) enthält, worin wenigstens einer der Reste R1, R2 und/oder R3 für eine C₁-C₆-Alkylgruppe steht.

Bei besonders geeigneten Verbindungen der Formel (I) steht einer der Reste ausgewählt aus R1, R2 und R3 für eine C₁-C₆-Alkylgruppe und die anderen beiden Reste ausgewählt aus R1, R2 und R3 für Wasserstoff.

In einer explizit ganz besonders bevorzugten Ausführungsform stehen R1 und R2 beide für ein Wasserstoffatom, und R3 steht für eine Methylgruppe.

Weiterhin werden besonders mit den Mitteln gute Färbeergebnisse erzielt, die mindestens eine Verbindung der Formel (I) enthalten, bei welcher die Reste R4, R5 und R6 unabhängig voneinander für eine C₁-C₆-Alkylgruppe oder eine Alkenylgruppe stehen. Insbesondere bevorzugt steht jeder der Reste R4, R5 und R6 für eine C₁-C₆-Alkylgruppe.

Ganz besonders bevorzugt ist es, wenn R4 und R5 beide für eine Methylgruppe stehen und R6 für eine Methylgruppe, eine Ethylgruppe oder eine n-Propylgruppe steht.

Weiterhin ist es ganz besonders bevorzugt, wenn R4 und R5 beide für eine Methylgruppe stehen und R6 für eine n-Propylgruppe steht.

Ein einer ebenfalls besonders bevorzugten Ausführungsform stehen die Reste R4, R5 und R6 jeweils für eine Methylgruppe.

Bei Verbindungen der Formel (I) besteht die Maßgabe, dass mindestens einer der Reste X, Y, Z für eine Gruppe N(R7)(R8) steht. Es konnten insbesondere dann Färbungen mit guten anwendungstechnischen Eigenschaften erhalten werden, wenn Verbindungen der Formel (I) eingesetzt wurden, bei denen X für eine Gruppe N(R7)(R8) steht und Y und Z jeweils für Wasserstoff stehen.

R7 und R8 stehen bevorzugt unabhängig voneinander für Wasserstoff oder eine C₁-C₆-Alkylgruppe. Besonders bevorzugt stehen R7 und R8 unabhängig voneinander für Wasserstoff oder für eine Methylgruppe. Verbindungen der Formel (I), bei denen sowohl R7 als auch R8 für Wasserstoff stehen, haben sich als besonders geeignet erwiesen und sind daher besonders bevorzugt.

Im Rahmen der zu dieser Erfindung führenden Arbeiten hat es sich als ganz besonders vorteilhaft herausgestellt, wenn X für eine Gruppe NH2 steht.

Eine weitere bevorzugte Ausführungsform ist daher ein Mittel zum Färben und gegebenenfalls Aufhellen von keratinischen Fasern, welches dadurch gekennzeichnet ist, dass es eine Verbindung der Formel (I) enthält, worin mindestens X für eine Gruppe NH₂ steht.

n steht bevorzugt für die Zahlen 2 oder 3 und ganz besonders bevorzugt für die Zahl 3.

A⁻ steht für ein physiologisch verträgliches Anion. Geeignete physiologisch verträgliche Anionen sind Halogenid, Hydrogensulfat, Sulfat, Benzolsulfonat, p-Toluolsulfonat, Acetat, Citrat, Lactat, Tartrat, Methylsulfat (H₃COSO₃⁻), Methylsulfoant oder Trifluormethansulfonat. Besonders bevorzugt steht A⁻ für Bromid oder für Methylsulfat (H₃COSO₃⁻), ganz besonders bevorzugt steht A⁻ für Methylsulfat (H₃COSO₃⁻).

Erfindungsgemäß bevorzugte Mittel zum Färben und gegebenenfalls gleichzeitigen Aufhellen keratinischer Fasern sind dadurch gekennzeichnet, dass sie mindestens eine Verbindung der allgemeinen Formel (I) enthalten, die ausgewählt ist aus
- 3-[(4-Amino-9,10-dihydro-3-methyl-9,10-dioxo-1-anthracenyl)amino]-N,N,N-trimethyl-1-propanaminiummethylsulfat,
- 3-[(4-Amino-9,10-dihydro-3-methyl-9,10-dioxo-1-anthracenyl)amino]-N,N,N-trimethyl-1-propanaminiumbromid,
- 3-[(4-Amino-9,10-dihydro-3-methyl-9,10-dioxo-1-anthracenyl)amino]-N,N,N-trimethyl-1-propanaminiumchlorid,
- 3-[(4-Amino-9,10-dihydro-3-methyl-9,10-dioxo-1-anthracenyl)amino]-N,N,N-trimethyl-1-propanaminium-p-toluolsulfonat,
- 3-[(4-Amino-9,10-dihydro-3-methyl-9,10-dioxo-1-anthracenyl)amino]-N,N,N-trimethyl-1-propanaminiumacetat,
- 3-{[9,10-Dihydro-4-(methylamino)-9,10-dioxo-1-anthracenyl]amino}-N,N-dimethyl-N-propyl-1-propanaminiummethylsulfat,
- 3-{[9,10-Dihydro-4-(methylamino)-9,10-dioxo-1-anthracenyl]amino}-N,N-dimethyl-N-propyl-1-propanaminiumbromid,
- 3-{[9,10-Dihydro-4-(methylamino)-9,10-dioxo-1-anthracenyl]amino}-N,N-dimethyl-N-propyl-1-propanaminiumchlorid,
- 3-{[9,10-Dihydro-4-(methylamino)-9,10-dioxo-1-anthracenyl]amino}-N,N-dimethyl-N-propyl-1-propanaminium-p-toluolsulfonat und
- 3-{[9,10-Dihydro-4-(methylamino)-9,10-dioxo-1-anthracenyl]amino}-N,N-dimethyl-N-propyl-1-propanaminiumacetat.

Optimal geeignet zur Lösung der erfindungsgemäßen Aufgabenstellung war die Verbindung der Formel (la), 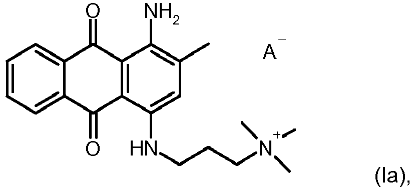 worin A⁻ für ein physiologisch verträgliches Anion, bevorzugt für Methylsulfat (H₃COSO₃⁻), steht. Die Verbindung (Ia), bei der A⁻ für Methylsulfat steht, ist auch unter der Bezeichnung Cationic Blue 347 bekannt.

Eine weitere besonders bevorzugte Ausführungsform ist daher ein Mittel zum Färben und gegebenenfalls Aufhellen von keratinischen Fasern, welches dadurch gekennzeichnet ist, dass es als Verbindung der Formel (I) die Verbindung gemäß Formel (Ia) enthält, 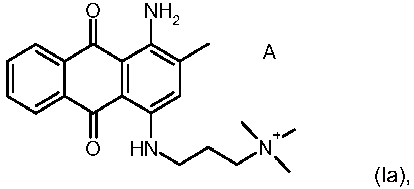 worin A⁻ für ein physiologisch verträgliches Anion, bevorzugt für Methylsulfat (H₃COSO₃⁻), steht.

Die erfindungsgemäßen Mittel zum Färben und gegebenenfalls gleichzeitigen Aufhellen von Keratinfasern enthalten die Verbindung(en) der Formel (I) vorzugsweise in Mengen oberhalb von 0,0001 Gew.-% und unterhalb von 5 Gew.-%, jeweils bezogen auf das gesamte Mittel.

Eine weitere bevorzugte Ausführungsform ist dabei ein Mittel, welches die Verbindung(en) der Formel (I) in einem Anteil von 0,0001 bis 5 Gew.-%, bevorzugt 0,005 bis 3,5 Gew.-%, besonders bevorzugt 0,01 bis 2,5 Gew.-%, insbesondere 0,05 bis 1,5 Gew.-%, und insbesondere bevorzugt von 0,01 bis 1,0 Gew.-%, jeweils bezogen auf das Gesamtgewicht des Mittels, enthält.

Als zweiten wesentlichen Formulierungsbestandteil (b) enthalten die erfindungsgemäßen Mittel mindestens ein Lecithin.

Lecithine im Sinne der vorliegenden Erfindung sind Mischungen von Phosphatestern von Fettsäuremonoglyceriden und Fettsäurediglyceriden, die weitere Gruppierungen am Glycerinkörper wie Enolether (Plasmensäure und deren Derivate), Ether (Plasmansäure und deren Derivate) oder am Phosphat wie Cholin (Phosphatidylcholin), Ethanolamin (Phosphatidylethanolamin), Serin (Phosphatidylserin), Inositol (Phosphatidylinositol) oder Glycerin (Phosphatidylglycerin) aufweisen können.

Bevorzugt wird erfindungsgemäß als Lecithin Soja-Lecithin oder gehärtetes (hydriertes) Soja-Lecithin eingesetzt.

Lecithine im Sinne der vorliegenden Erfindung umfassen auch synthetische, biomimetische Phospholipide. Eine weitere bevorzugte Ausführungsform des ersten Erfindungsgegenstands ist daher ein Mittel, welches als Lecithin mindestens ein biomimetisches Phospholipid enthält.

Beispiele für biomimetische Phospholipide sind Verbindungen der Formel (II), 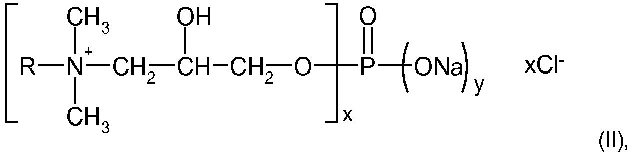 worin R für einen Fettsäureacylamido(C₂-C₆)alkyl-Rest steht, x für die Zahl 1 oder 2 steht und die Summe aus x+y = 3 ist.

Beispiele für geeignete Fettsäureacylamido(C₂-C₆)alkyl-Reste sind Cocamidoethyl, Cocamidopropyl, Oleamidoethyl, Oleamidopropyl, Linoleamidoethyl, Linoleamidopropyl, Cetearamidoethyl, Cetearamidopropyl, Behenamidoethyl, Behenamidopropyl, Stearamidopropyl und Stearamidoethyl.

Besonders bevorzugt steht R für Cocamidopropyl- und/oder Linoleamidopropyl-Reste. Solche Verbindungen der Formel (II) werden unter der INCI-Bezeichnung Cocamidopropyl PG-Dimonium Chloride Phosphate (Handelsname: Phospholipid PTC) und Linoleamidopropyl PG-Dimonium Chloride Phosphate (Handelsname: Phospholipid EFA) vertrieben.

Eine weitere bevorzugte Ausführungsform des ersten Erfindungsgegenstands ist daher ein Mittel, welches als Lecithin Linoleamidopropyl PG-Dimonium Chloride Phosphate und/oder Cocamidopropyl PG-Dimonium Chloride Phosphate enthält.

Die Lecithine sind im Mittel bevorzugt in einem bestimmten Anteil vorhanden, der eine optimale Pflegeleistung der Haare garantiert und die Farbkonservierung auf der Faser maximal unterstützt.

Eine weitere Ausführungsform des ersten Erfindungsgegenstands ist daher ein Mittel, welches dadurch gekennzeichnet ist, dass das oder die Lecithin(e) in einem Anteil von 0,001 bis 5 Gew.-%, bevorzugt 0,005 bis 3 Gew.-%, besonders bevorzugt 0,01 bis 1,5 Gew.-%, insbesondere 0,05 bis 1,0 Gew.-%, und insbesondere bevorzugt von 0,1 bis 0,5 Gew.-%, jeweils bezogen auf das Gesamtgewicht des Mittels, enthält.

In einer weiteren bevorzugten Ausführungsform enthalten die erfindungsgemäßen Mittel neben der Verbindung der Formel (I) zusätzlich mindestens einen weiteren direktziehenden Farbstoff. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen. Die zusätzlichen direktziehenden Farbstoffe werden jeweils bevorzugt in einem Anteil von 0,001 bis 2 Gew.-%, bezogen auf die gesamte Anwendungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17 sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Färbeergebnisse mit herausragender Farbintensität, Brillanz und guter Waschechtheit werden insbesondere dann erhalten, wenn die erfindungsgemäßen Mittel als weiteren direktziehenden Farbstoff mindestens einen Farbstoff ausgewählt aus D&C Red No. 33 (Acid Red 33), Acid Black No. 1, D&C Orange No. 4 (Acid Orange No. 4), Acid Red 18, Basic Red 76, Acid Violet 43, HC Blue No. 12, N-(2-Hydroxethyl)-4-methyl-2-nitroanilin (Methyl Yellow), HC Yellow No. 2, Red B 54 und 2-Amino-6-chlor-4-phenol enthalten.

Es ist nicht erforderlich, dass die fakultativ enthaltenen direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Die erfindungsgemäßen Mittel können weiterhin auch als Oxidationsfärbemittel eingesetzt werden. Solche Oxidationsfärbemittel enthalten zusätzlich mindestens ein Oxidationsfarbstoffvorprodukt, bevorzugt zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)-propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)-phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Die direktziehenden Farbstoffe, Entwicklerkomponenten und Kupplerkomponenten werden bevorzugt jeweils in einer Menge von 0,0001 bis 5,0 Gew.-%, vorzugsweise 0,001 bis 2,5 Gew.-%, jeweils bezogen auf das anwendungsbereite Mittel, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Im Falle von Oxidationsfärbemitteln enthalten die Mittel bevorzugt ein Oxidationsmittel, bevorzugt Wasserstoffperoxid. Die Mengen an Wasserstoffperoxid entsprechen den Mengen in den erfindungsgemäßen Aufhellmitteln.

Die Mittel können weiterhin als aufhellende Färbemittel eingesetzt werden. Zur Erzielung des Aufhelleffekts enthalten die Mittel dazu Wasserstoffperoxid und/oder eines seiner festen Anlagerungsprodukte an organische oder anorganische Verbindungen.

Eine weitere Ausführungsform des ersten Erfindungsgegenstands ist daher dadurch gekennzeichnet, dass das Mittel zusätzlich Wasserstoffperoxid und/oder eines seiner festen Anlagerungsprodukte an organische oder anorganische Verbindungen enthält.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte anwendungsbereite Mittel des ersten Erfindungsgegenstands sind dadurch gekennzeichnet, dass sie, bezogen auf das Gesamtgewicht des anwendungsbereiten Mittels, 0,5 bis 20 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht des Mittels, enthalten.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, in dem erfindungsgemäßen Mittel enthalten.

Eine weitere bevorzugte Ausführungsform ist ein Mittel zum Färben und gegebenenfalls Aufhellen keratinischer Fasern, welches zusätzlich Wasserstoffperoxid, eines seiner festen Anlagerungsprodukte an organische oder anorganische Verbindungen, Ammoniumperoxodisulfat, Kaliumperoxodisulfat und/oder Natriumperoxodisulfat jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, enthält.

Das Mittel kann zur Verstärkung der Blondierwirkung weitere Bleichkraftverstärker enthalten, wie beispielsweise Tetraacetylethylendiamin (TAED), 1,5-Diacetyl-2,4-dioxohexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluolsulfonat, 2-Acetyl-1-methylpyridinium-p-toluolsulfonat, sowie N-Methyl-3,4-dihydroisochinolinium-p-toluolsulfonat.

Zur weiteren Steigerung der Aufhellung kann der erfindungsgemäßen Zusammensetzung zusätzlich mindestens eine SiO₂-Verbindung, wie Kieselsäure oder Silicate, insbesondere Wassergläser, zugesetzt sein. Es kann erfindungsgemäß bevorzugt sein, die SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Die anwendungsbereiten Färbemittel können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die anwendungsbereiten Färbemittel als flüssige Zubereitung bereitgestellt und den Mitteln daher zusätzlich eine weitere oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen, nichtionischen, amphoteren und kationischen Tensiden ausgewählt.

Erfindungsgemäß bevorzugte Mittel sind dadurch gekennzeichnet, dass das Mittel mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die zusätzlichen anionischen Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugte Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Als nichtionische Tenside eignen sich insbesondere Anlagerungsprodukte von Ethylenoxid und/oder Propylenoxid an Fettalkohole, Fettsäuren, Fettsäureamide, Polyolester von Fettsäuren sowie und Polyolether von Fettalkoholen sowie Alkylpolyglucoside. Beispiele für geeignete nichtionische Tenside sind Laureth-2, Beheneth-10, Ceteareth-12, Trideceth-12, Oleth-16, Ceteareth-20, Ceteareth-30 und Ceteareth-50 sowie PPG-1 Trideceth-6, PEG-7 Oleate, PEG-90 Stearate, PEG-30 Cocoate, Polysorbate-20, Polysorbate-60, Polysorbate-65, Polysorbate-80, Polysorbate-85, Lauryl Glucoside, Decyl Glucoside und/oder Coco Glucoside.

Erfindungsgemäß bevorzugte Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Die nichtionischen, zwitterionischen oder amphoteren Tenside werden in Anteilen von 0,01 bis 45 Gew.-%, bevorzugt 0,1 bis 30 Gew.-% und ganz besonders bevorzugt von 1 bis 15 Gew.-%, bezogen auf die Gesamtmenge der anwendungsbereiten Mittel, eingesetzt.

Erfindungsgemäß geeignete Mittel können auch kationische Tenside vom Typ der quartären Ammoniumverbindungen, der Esterquats und der Amidoamine enthalten. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus der Amidoamine stellt das unter der Bezeichnung Tegoamid® S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß verwendeten Mitteln bevorzugt in Anteilen von 0,05 bis 10 Gew.-%, bezogen auf das gesamte Mittel, enthalten.

Die anwendungsbereiten Mittel können weitere Hilfs- und Zusatzstoffe enthalten. So hat es sich als vorteilhaft erwiesen, wenn die Mittel mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen.

### Geeignete Verdickungsmittel sind

- kationische, synthetische Polymere;
- anionische, synthetische Polymere, wie Polyacrylate, Acrylates Copolymer, Copolymere von Acrylsäure und Methacrylsäure
- natürlich vorkommende Verdickungsmittel, wie nichtionische Guargums, Skleroglucangums oder Gummi arabicum, Ghatti-Gummi, Karaya-Gummi, Tragant-Gummi, Carrageen-Gummi, Johannisbrotkernmehl, Pektine, Xanthane, Alginate, Stärke-Fraktionen und Derivate wie Amylose, Amylopektin und Dextrine, sowie Cellulosederivate, wie beispielsweise Carboxymethylcellulose Methylcellulose und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Färbeprozesse auf Keratinfasern laufen üblicherweise im schwach sauren bis alkalischen Bereich, bevorzugt im schwach sauren bis schwach alkalischen Milieu ab. Um die Keratinfasern und auch die Haut so weit wie möglich zu schonen, ist die Einstellung eines zu hohen pH-Wertes jedoch nicht wünschenswert. Der pH-Wert der erfindungsgemäßen Mittel kann daher zwischen 3 und 11, bevorzugt zwischen 5 und 8 liegen. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel werden bevorzugt aus Ammoniak, Alkanolaminen, basischen Aminosäuren sowie anorganischen Alkalisierungsmitteln ausgewählt. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin.. Zur Einstellung des pH-Werts verwendbare Acificierungsmittel sind organische Säuren, wie Citronensäure, Essigsäure, Ascorbinsäure, Benzoesäure, Milchsäure, Äpfelsäure und Maleinsäure, sowie mineralische Säuren, wie Salzsäure, Schwefelsäure oder Phosphorsäure.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazoliniummethochlorid-Copolymere und quaternierter Polyvinylalkohol, insbesondere Polyquaternium-2, Polyquaternium-4, Polyquaternium-6, Polyquaternium-7, Polyquaternium-10, Polyquaternium-11, Polyquaternium-16, Polyquaternium-24, Polyquaternium-28, Polyquaternium-37, Polyquaternium-44, Polyquaternium-46, Polyquaternium-55, Polyquaternium-67, Polyquaternium-68, Polyquaternium-69 und Polyquaternium-87; zwitterionische und amphotere Polymere, wie insbesondere Polyquaternium-22 und Polyquaternium-39; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und - distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Zur Anwendung der erfindungsgemäßen Mittel eignet sich insbesondere ein Verfahren zum Färben und gegebenenfalls Aufhellen von keratinischen Fasern, insbesondere menschlichen Haaren, welches dadurch gekennzeichnet ist, dass ein Mittel des ersten Erfindungsgegenstands auf die keratinhaltigen Fasern aufgebracht, 5 bis 60 Minuten auf der Faser belassen und anschließend mit Wasser wieder ausgespült oder mit einem Shampoo ausgewaschen wird. Bevorzugt beträgt die Einwirkzeit der anwendungsbereiten Färbemittel 5 bis 45 min, insbesondere 10 bis 40 min, besonders bevorzugt 15 bis 35 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie z.B. warme Luft eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Färbemittel einen stark tensidhaltigen Träger besitzt.

Die erfindungsgemäßen Mittel können als Einkomponentenmittel (Färbe- und Aufhellmittel) oder als Mehrkomponentenmittel wie Zweikomponenten-Mittel oder Dreikomponenten-Mittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Enthält das erfindungsgemäße Mittel sowohl direktziehende Farbstoffe - sowie gegebenenfalls zusätzlich Oxidationsfarbstoffvorprodukte - als auch Oxidationsmittel, so werden diese, um eine vorzeitige, unerwünschte Reaktion miteinander zu verhindern, zweckmäßigerweise getrennt voneinander konfektioniert und erst unmittelbar vor der Anwendung in Kontakt gebracht.

Ein Färbeverfahren, bei dem die Färbecreme und das Oxidationsmittel zunächst getrennt vorliegen, ist daher bevorzugt. Ein weiterer Gegenstand der vorliegenden Erfindung ist daher ein Verfahren zum Färben und Aufhellen von menschlichen Haaren, bei dem eine Zusammensetzung auf wässriger Grundlage, enthaltend Wasserstoffperoxid, mit einem erfindungsgemäßen Mittel enthaltend mindestens eine Verbindung der Formel (I) zu einer homogenen Zusammensetzung vermischt, und diese auf das Haar aufgebracht wird. Das Lecithin (b) kann in diesem Fall sowohl zusammen mit der Wasserstoffperoxid-Lösung als auch mit der Verbindung der Formel (I) konfektioniert werden.

In einer weiteren Ausführungsform der vorliegenden Erfindung sind daher Mittel bevorzugt, welche dadurch gekennzeichnet sind, dass sie unmittelbar vor der Anwendung durch Vermischen mindestens zweier Zubereitungen hergestellt werden, wobei die mindestens zwei Zubereitungen in mindestens zwei getrennt konfektionierten Containern bereitgestellt werden, und wobei ein Container ein Mittel (A), welches in einem kosmetischen Träger mindestens einen kationischen Anthrachinonfarbstoff der Formel (I) und mindestens ein Lecithin (b) - sowie gegebenenfalls zusätzlich Oxidationsfarbstoffvorprodukte enthält, und ein weiterer Container eine Oxidationsmittelzubereitung (B), enthaltend mindestens ein Oxidationsmittel, enthält.

Die Formulierung einer Kombination von (a) Verbindungen der allgemeinen Formel (I) mit (b) Lecithin eignet sich hervorragend zur Erzeugung von intensiven Färbungen mit hoher Brillanz, hohem Glanz und einer niedrigen Selektivität in Verbindung mit einer hervorragenden Waschechtheit.

Bezüglich weiterer bevorzugter Ausführungsformen des erfindungsgemäßen Verfahren gilt mutatis mutandis das zu den erfindungsgemäßen Mitteln Gesagte.

### Beispiele

| **Formulierungsbeispiel 1** | **Gew.-%** |
|---|---|
| Phospholipid EFA | 0,50 |
| Methylparaben | 0,15 |
| Propylparaben | 0,15 |
| Dehyquart A CA | 5,00 |
| Cetyl alcohol | 4,00 |
| HC Blue No. 12 | 0,5 |
| Bluequat Bromide | 0,20 |
| N-(2-Hydroxyethyl)-4-methyl-2-nitroanilin (Methyl Yellow) | 0,05 |
| HC Yellow No. 2 | 0,08 |
| Red B 54 | 0,06 |
| 2-Amino-6-chloro-4-phenol | 0,08 |
| HC Yellow No. 13 | 0,07 |
| Cationic Blue 347 | 0,50 |
| Procetyl AWS-LQ | 1,00 |
| Monoethanolamine | 0,25 |
| Parfüm | qs |
| Wasser | Ad 100 |

| | |
|---|---|
| Dehyquart A CA ca. 25% Aktivgehalt; INCI-Bezeichnung: Aqua, Cetrimonium Chloride (BASF) Phospholipid EFA INCI-Bezeichnung: Linoleamidopropyl PG-Dimonium Chloride Phosphate (30%), Propylene Glycol (25%) (Uniquema) Procetyl AWS-LQ INCI-Bezeichnung: PPG-5 Ceteth-20 (Croda) | |

## Patentansprüche

1. Mittel zum Färben und/oder Aufhellen von keratinischen Fasern, insbesondere menschlichen Haaren, **dadurch gekennzeichnet, dass** es in einem kosmetischen Träger
(a) mindestens eine Verbindung der Formel (I) 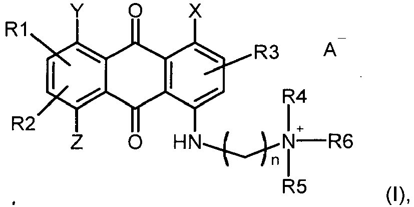 worin
R1, R2, R3 jeweils unabhängig voneinander für Wasserstoff, Halogen, eine Nitrogruppe, eine Cyanogruppe, eine Carboxylgruppe, eine Sulfonsäuregruppe, eine Hydroxygruppe, eine C₁-C₆-Acylaminogruppe, eine Carboxamidgruppe, eine Sulfonamidgruppe, eine C₁-C₆-Alkylgruppe, C₁-C₆-Alkoxygruppe, eine C₂-C₆-Alkenylgruppe, für eine C₂-C₆-Hydroxyalkylgruppe oder eine C₁-C₆-Alkoxy-C₂-C₆-Alkylgruppe stehen;
R4, R5, R6 jeweils unabhängig voneinander für eine C₁-C₆-Alkylgruppe, eine C₂-C₆-Alkenylgruppe, für eine C₂-C₆-Hydroxyalkylgruppe oder eine C₁-C₆-Alkoxy- C₂-C₆-Alkylgruppe stehen;
X, Y, Z jeweils unabhängig voneinander für Wasserstoff oder eine Gruppe N(R7)(R8) stehen,
worin
R7 und R8 jeweils unabhängig voneinander für Wasserstoff, eine C₁-C₆-Alkylgruppe, eine C₂-C₆-Alkenylgruppe, für eine C₂-C₆-Hydroxyalkylgruppe oder eine C₁-C₆-Alkoxy- C₂-C₆-Alkylgruppe stehen,
unter der Maßgabe, dass mindestens einer der Reste X, Y, Z für eine Gruppe N(R7)(R8) steht,
n für eine Zahl von 2 bis 6 steht
A⁻ für ein physiologisch verträgliches Anion steht, und
(b) mindestens ein Lecithin enthält.

2. Mittel nach Anspruch 1, **dadurch gekennzeichnet, dass** es eine Verbindung der Formel (I) enthält, worin wenigstens einer der Reste R1, R2 und/oder R3 für eine C₁-C₆-Alkylgruppe steht.

3. Mittel nach Anspruch 1 oder 2, **dadurch gekennzeichnet, dass** es eine Verbindung der Formel (I) enthält, worin mindestens X für eine Gruppe NH₂ steht.

4. Mittel nach einem der Ansprüche 1 bis 3, **dadurch gekennzeichnet, dass** es als Verbindung der Formel (I) die Verbindung gemäß Formel (Ia), 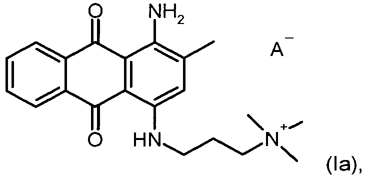 worin A⁻ für ein physiologisch verträgliches Anion steht, enthält.

5. Mittel nach einem der Ansprüche 1 bis 4, **dadurch gekennzeichnet, dass** es die Verbindung(en) gemäß Formel (I) in einem Anteil von 0,0001 bis 5 Gew.-%, bevorzugt 0,005 bis 3,5 Gew.-%, besonders bevorzugt 0,01 bis 2,5 Gew.-%, insbesondere 0,05 bis 1,5 Gew.-%, und insbesondere bevorzugt von 0,01 bis 1,0 Gew.-%, jeweils bezogen auf das Gesamtgewicht des Mittels, enthält.

6. Mittel nach einem der Ansprüche 1 bis 5, **dadurch gekennzeichnet, dass** es als Lecithin mindestens ein biomimetisches Phospholipid enthält.

7. Mittel nach einem der Ansprüche 1 bis 6, **dadurch gekennzeichnet, dass** es als Lecithin Linoleamidopropyl PG-Dimonium Chloride Phosphate und/oder Cocamidopropyl PG-Dimonium Chloride Phosphate enthält.

8. Mittel nach einem der Ansprüche 1 bis 7, **dadurch gekennzeichnet, dass** es das oder die Lecithin(e) in einem Anteil von 0,001 bis 5 Gew.-%, bevorzugt 0,005 bis 3 Gew.-%, besonders bevorzugt 0,01 bis 1,5 Gew.-%, insbesondere 0,05 bis 1,0 Gew.-%, und insbesondere bevorzugt von 0,1 bis 0,5 Gew.-%, jeweils bezogen auf das Gesamtgewicht des Mittels, enthält.

9. Mittel nach einem der Ansprüche 1 bis 8, **dadurch gekennzeichnet, dass** es neben der Verbindung der Formel (I) zusätzlich mindestens einen weiteren direktziehenden Farbstoff aus der Gruppe Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau enthält.

10. Mittel nach einem der Ansprüche 1 bis 9, **dadurch gekennzeichnet, dass** es neben der Verbindung der Formel (I) zusätzlich mindestens einen weiteren direktziehenden Farbstoff aus der Gruppe Basic Blue 7, Basic Blue 26, Basic Violet 2, Basic Violet 14, Basic Yellow 57, Basic Red 76, Basic Blue 99, Basic Brown 16, Basic Brown 17, Basic Yellow 87, Basic Orange 31 und Basic Red 51 enthält.

11. Mittel nach einem der Ansprüche 1 bis 10, **dadurch gekennzeichnet, dass** es neben der Verbindung der Formel (I) zusätzlich mindestens einen weiteren direktziehenden Farbstoff aus der Gruppe HC Yellow 2, HC Yellow 4, HC Yellow 5, HC Yellow 6, HC Yellow 12, HC Orange 1, Disperse Orange 3, HC Red 1, HC Red 3, HC Red 7, HC Red 10, HC Red 11, HC Red 13, HC. Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)-amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol enthält.

12. Mittel nach einem der Ansprüche 1 bis 11, **dadurch gekennzeichnet, dass** es zusätzlich mindestens ein kationisches, synthetisches Polymer als Verdickungsmittel enthält.

13. Mittel nach einem der Ansprüche 1 bis 12, **dadurch gekennzeichnet, dass** sein pH-Wert zwischen 5 und 8 liegt.

14. Verfahren zum Färben menschlicher Haare, umfassend die Schritte:
A. Auftragen eines Mittel nach einem der Ansprüche 1 bis 13 auf das Haar;
B. Einwirken des Mittels für einen Zeitraum von 5 bis 45 Minuten, bevorzugt von 10 bis 35 Minuten, auf dem Haar;
C. Ausspülen des Haares.

## Claims

1. An agent for dyeing and/or lightening keratin fibers, in particular human hair, **characterized in that** it contains in a cosmetic carrier
(a) at least one compound of formula (I) 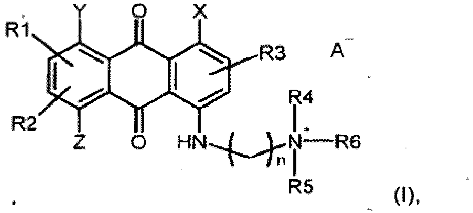 where
R1, R2, R3 each represent, independently of one another, hydrogen, halogen, a nitro group, a cyano group, a carboxyl group, a sulfonic acid group, a hydroxy group, a C₁-C₆ acyl amino group, a carboxamide group, a sulfonamide group, a C₁-C₆ alkyl group, C₁-C₆ alkoxy group, a C₂-C₆ alkenyl group, a C₂-C₆ hydroxyalkyl group or a C₁-C₆ alkoxy-C₂-C₆ alkyl group;
R4, R5, R6 each represent, independently of one another, a C₁-C₆ alkyl group, a C₂-C₆ alkenyl group, a C₂-C₆ hydroxyalkyl group or a C₁-C₆ alkoxy-C₂-C₆ alkyl group;
X, Y, Z each represent, independently of one another, hydrogen or an N(R7)(R8) group,
where
R7 and R8 each represent, independently of one another, hydrogen, a C₁-C₆ alkyl group, a C₂-C₆ alkenyl group, a C₂-C₆ hydroxyalkyl group or a C₁-C₆ alkoxy-C₂-C₆ alkyl group,
with the proviso that at least one of the functional groups X, Y, Z represents an N(R7)(R8) group;
n represents a number from 2 to 6;
A⁻ represents a physiologically acceptable anion, and
(b) at least one lecithin.

2. The agent according to claim 1, **characterized in that** it contains a compound of formula (I), where at least one of the functional groups R1, R2 and/or R3 represents a C₁-C₆ alkyl group.

3. The agent according to claim 1 or 2, **characterized in that** it contains a compound of formula (I), where at least X represents an NH₂ group.

4. The agent according to one of claims 1 to 3, **characterized in that** it contains the compound according to formula (la) as the compound of formula (I) 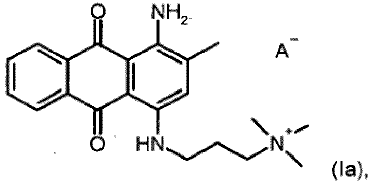 where A⁻ represents a physiologically acceptable anion.

5. The agent according to one of claims 1 to 4, **characterized in that** it contains the compound(s) according to formula (I) in a proportion of from 0.0001 to 5 wt.%, preferably from 0.005 to 3.5 wt.%, particularly preferably from 0.01 to 2.5 wt.%, in particular from 0.05 to 1.5 wt.%, and more particularly preferably from 0.01 to 1.0 wt.%, in each case based on the total weight of the agent.

6. The agent according to one of claims 1 to 5, **characterized in that** it contains at least one biomimetic phospholipid as the lecithin.

7. The agent according to one of claims 1 to 6, **characterized in that** it contains linoleamidopropyl PG-dimonium chloride phosphate and/or cocamidopropyl PG-dimonium chloride phosphate as the lecithin.

8. The agent according to one of claims 1 to 7, **characterized in that** it contains the lecithin(s) in a proportion of from 0.001 to 5 wt.%, preferably from 0.005 to 3 wt.%, particularly preferably from 0.01 to 1.5 wt.%, in particular from 0.05 to 1.0 wt.%, and more particularly preferably from 0.1 to 0.5 wt.%, in each case based on the total weight of the agent.

9. The agent according to one of claims 1 to 8, **characterized in that** it also contains, in addition to the compound of formula (I), at least one further direct dye from the group Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, bromophenol blue and tetrabromophenol blue.

10. The agent according to one of claims 1 to 9, **characterized in that** it also contains, in addition to the compound of formula (I), at least one further direct dye from the group Basic Blue 7, Basic Blue 26, Basic Violet 2, Basic Violet 14, Basic Yellow 57, Basic Red 76, Basic Blue 99, Basic Brown 16, Basic Brown 17, Basic Yellow 87, Basic Orange 31 and Basic Red 51.

11. The agent according to one of claims 1 to 10, **characterized in that** it also contains, in addition to the compound of formula (I), at least one further direct dye from the group HC Yellow 2, HC Yellow 4, HC Yellow 5, HC Yellow 6, HC Yellow 12, HC Orange 1, Disperse Orange 3, HC Red 1, HC Red 3, HC Red 7, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-diamino-2-nitrobenzene, 2-amino-4-nitrophenol, 1,4-bis(2-hydroxyethyl)-amino-2-nitrobenzene, 3-nitro-4-(2-hydroxyethyl)aminophenol, 2-(2-hydroxyethyl)amino-4,6-dinitrophenol, 4-[(2-hydroxyethyl)amino]-3-nitro-1-methylbenzene, 1-amino-4-(2-hydroxyethyl)amino-5-chloro-2-nitrobenzene, 4-amino-3-nitrophenol, 1-(2'-ureidoethyl)amino-4-nitrobenzene, 2-[(4-amino-2-nitrophenyl)amino]benzoic acid, 4-[(3-hydroxypropyl)amino]-3-nitrophenol, 4-nitro-o-phenylenediamine, 6-nitro-1,2,3,4-tetrahydroquinoxaline, 2-hydroxy-1,4-naphthoquinone, picramic acid and salts thereof, 2-amino-6-chloro-4-nitrophenol, 4-ethylamino-3-nitrobenzoic acid, and 2-chloro-6-ethylamino-4-nitrophenol.

12. The agent according to one of claims 1 to 11, **characterized in that** it also contains at least one cationic, synthetic polymer as a thickener.

13. The agent according to one of claims 1 to 12, **characterized in that** its pH is between 5 and 8.

14. A method for dyeing human hair, comprising the steps of:
A. applying an agent according to one of claims 1 to 13 to the hair;
B. allowing the agent to act on the hair for a period of from 5 to 45 minutes, preferably from 10 to 35 minutes;
C. rinsing the hair.

## Revendications

1. Agent de coloration et/ou d'éclaircissement de fibres kératiniques, en particulier de cheveux humains, **caractérisé en ce qu'**il contient dans un agent cosmétique
(a) au moins un composé de formule (I) 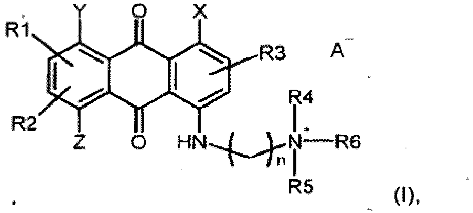 dans lequel
R1, R2, R3 représentent chacun indépendamment les uns des autres l'hydrogène, l'halogène, un groupe nitro, un groupe cyano, un groupe carboxyle, un groupe acide sulfonique, un groupe hydroxy, un groupe acylamino en C₁-C₆, un groupe carboxamide, un groupe sulfonamide, un groupe alkyle en C₁-C₆, un groupe alcoxy en C₁-C₆, un groupe alcényle en C₂-C₆, un groupe hydroxyalkyle en C₂-C₆ ou un groupe alcoxy en C₁-C₆-alkyle en C₂-C₆ ;
R4, R5, R6 représentent chacun indépendamment les uns des autres un groupe alkyle en C₁-C₆, un groupe alcényle en C₂-C₆, un groupe hydroxyalkyle en C₂-C₆ ou un groupe alcoxy en C₁-C₆-alkyle en C₂-C₆ ;
X, Y, Z représentent chacun indépendamment les uns des autres l'hydrogène ou un groupe N(R7)(R8),
dans lequel
R7 et R8 représentent chacun indépendamment les uns des autres l'hydrogène, un groupe alkyle en C₁-C₆, un groupe alcényle en C₂-C₆, un groupe hydroxyalkyle en C₂-C₆ ou un groupe alcoxy en C₁-C₆-alkyle en C₂-C₆,
à condition qu'au moins un des radicaux X, Y, Z représente un groupe N(R7)(R8),
n représente un nombre allant de 2 à 6,
A⁻ représente un anion physiologiquement compatible et
(b) contient au moins une lécithine.

2. Agent selon la revendication 1, **caractérisé en ce qu'**il contient un composé de formule (I), dans laquelle au moins un des radicaux R1, R2 et/ou R3 représente un groupe alkyle en C₁-C₆.

3. Agent selon la revendication 1 ou 2, **caractérisé en ce qu'**il contient un composé de formule (I), dans laquelle au moins X représente un groupe NH₂.

4. Agent selon l'une des revendications 1 à 3, **caractérisé en ce qu'**il contient, comme composé de formule (I), le composé selon la formule (Ia), 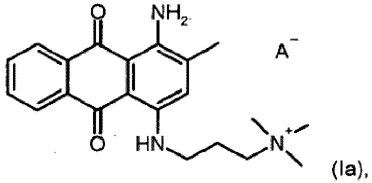 dans laquelle A⁻ représente un anion physiologiquement compatible.

5. Agent selon l'une des revendications 1 à 4, **caractérisé en ce qu'**il contient le ou les composé(s) selon la formule (I) dans une proportion de 0,0001 à 5 % en poids, de préférence de 0,005 à 3,5 % en poids, de manière davantage préférée de 0,01 à 2,5 % en poids, en particulier de 0,05 à 1,5 % en poids, et de manière particulièrement préférée de 0,01 à 1,0 % en poids, respectivement rapporté au poids total de l'agent.

6. Agent selon l'une des revendications 1 à 5, **caractérisé en ce qu'**il contient, comme lécithine, au moins un phospholipide biomimétique.

7. Agent selon l'une des revendications 1 à 6, **caractérisé en ce qu'**il contient, comme lécithine, au moins du linoleamidopropyl PG-dimonium chloride phosphate et/ou cocamidopropyl PG-dimonium chloride phosphate.

8. Agent selon l'une des revendications 1 à 7, **caractérisé en ce qu'**il contient la ou les lécithine(s) dans une proportion de 0,001 à 5 % en poids, de préférence de 0,005 à 3 % en poids, de manière davantage préférée de 0,01 à 1,5 % en poids, en particulier de 0,05 à 1,0 % en poids, et de manière particulièrement préférée de 0,1 à 0,5 % en poids, respectivement rapporté au poids total de l'agent.

9. Agent selon l'une des revendications 1 à 8, **caractérisé en ce qu'**il contient, en plus du composé de formule (I), au moins un colorant à action directe supplémentaire issu de l'ensemble comprenant Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, bleu de bromophénol et bleu de tétrabromophénol.

10. Agent selon l'une des revendications 1 à 9, **caractérisé en ce qu'**il contient, en plus du composé de formule (I), au moins un colorant à action directe supplémentaire issu de l'ensemble comprenant Basic Blue 7, Basic Blue 26, Basic Violet 2, Basic Violet 14, Basic Yellow 57, Basic Red 76, Basic Blue 99, Basic Brown 16, Basic Brown 17, Basic Yellow 87, Basic Orange 31 et Basic Red 51.

11. Agent selon l'une des revendications 1 à 10, **caractérisé en ce qu'**il contient, en plus du composé de formule (I), au moins un colorant à action directe supplémentaire issu de l'ensemble comprenant HC Yellow 2, HC Yellow 4, HC Yellow 5, HC Yellow 6, HC Yellow 12, HC Orange 1, Disperse Orange 3, HC Red 1, HC Red 3, HC Red 7, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, le 1,4-diamino-2-nitrobenzène, le 2-amino-4-nitrophénol, le 1,4-bis-(2-hydroxyéthyl)-amino-2-nitrobenzène, le 3-nitro-4-(2-hydroxyéthyl)-aminophénol, le 2-(2-hydroxyéthyl)amino-4,6-dinitrophénol, le 4-[(2-hydroxyéthyl)amino]-3-nitro-1-méthylbenzène, le 1-amino-4-(2-hydroxyéthyl)-amino-5-chloro-2-nitrobenzène, le 4-amino-3-nitrophénol, le 1-(2'-uréidoéthyl)amino-4-nitrobenzène, l'acide 2-[(4-amino-2-nitrophényl)amino]-benzoïque, le 4-[(3-hydroxypropyl)amino]-3-nitrophénol, la 4-nitro-o-phénylènediamine, la 6-nitro-1,2,3,4-tétrahydroquinoxaline, la 2-hydroxy-1,4-naphtoquinone, l'acide picramique et ses sels, le 2-amino-6-chloro-4-nitrophénol, l'acide 4-éthylamino-3-nitrobenzoïque et le 2-chloro-6-éthylamino-4-nitrophénol.

12. Agent selon l'une des revendications 1 à 11, **caractérisé en ce qu'**il contient au moins un polymère synthétique cationique supplémentaire comme agent épaississant.

13. Agent selon l'une des revendications 1 à 12, **caractérisé en ce que** son pH est compris entre 5 et 8.

14. Procédé de coloration des cheveux humains, comprenant les étapes suivantes :
A. Application d'un agent selon l'une des revendications 1 à 13 sur les cheveux ;
B. Action de l'agent pendant une durée de 5 à 45 minutes, de préférence de 10 à 35 minutes, sur les cheveux ;
C. Rinçage des cheveux.
